# EUROPEAN PATENT APPLICATION

(11) **EP 0 870 753 A1**
(43) Date of publication of application: **14.10.1998**
(21) Application number: 97302326.0
(22) Date of filing: 04.04.1997
(51) Int. Cl.: C07C 67/38, C07C 69/54

(54) **A process for the preparation of methacrylate esters**

(71) Applicant: IMPERIAL CHEMICAL INDUSTRIES PLC, London SW1P 3JF (GB)
(72) Inventor: The designation of the inventor has not yet been filed
(74) Representative: Nevard, Edward John

(57) **Abstract**

A process for the preparation of methacrylate esters, wherein (a) propyne is stripped in a distillation column from a solvent stream containing dissolved propyne, to afford a gaseous propyne stream that is subsequently condensed, and (b) the condensed propyne is contacted with carbon monoxide and an alcohol in the presence of a carbonylation catalyst, characterised in that the propyne is stripped in a distillation column that is equipped with an internal condenser to afford partial condensation for the supply of reflux.

## Description

The invention relates to a process for the preparation of methacrylate esters. More in particular, the invention relates to a process for preparing methyl methacrylate (MMA) in a safe manner.

Methacrylate esters may be produced by contacting a propyne feed, isolated from a C₃-hydrocarbon feed, with carbon monoxide and an alcohol in the presence of a carbonylation catalyst. As propadiene tends to poison the carbonylation catalyst, it is selectively removed, typically by extractive distillation.

Thus, in EP-A-0,392,601 a process is disclosed for the preparation of an alkyl methacrylate, which comprises: (a) selectively removing propadiene from a C₃-hydrocarbon feed comprising a mixture of propyne and propadiene to afford a propyne feed in which the ratio of propyne to propadiene is at least 6, and (b) contacting the propyne feed with carbon monoxide and an alcohol in the presence of a carbonylation catalyst. Typically, the propyne feed is produced by stripping a solvent stream containing dissolved propyne, that has been used in the extractive distillation of propadiene. After being condensed and collected, the propyne is stored in a buffer, from which it is withdrawn to feed the reaction. This process has been discussed in the SRI PEP Report 11D of January 1993 ("METHACRYLIC ACID AND ESTERS") and a process flow diagram is provided in Figure 9.1 thereof. Due to the presence of pure liquid propyne, this process calls for stringent safety precautions.

In EP-A-0,533,291 this problem of having to handle pure liquid propyne is addressed to some extent. That invention concerns the replacement of the propyne buffer by a storage facility wherein the propyne is stored in admixture with the alcohol it is to be reacted with. This mitigates safety requirements and improves the compatibility of the methacrylate ester production with refinery operations. However, the distillation column wherein the propyne is stripped also requires a buffer: the reflux and product accumulator drum that is used together with the (total) overhead condenser. A large portion of the condensed propyne that is accumulated in this drum is pumped back to the column for reflux, and only a minor portion of this condensate is collected as the product stream (typically the column operates at a reflux ratio of 2:1). Replacement of this drum by a storage facility wherein the condensed propyne is stored in admixture with the alcohol would result in the introduction of the alcohol into the distillation column. This contamination of the reflux is not desirable. The size of the drum typically is in the range of 1 to 2 m³; providing a 5 minute control volume for a column operating at an overhead flow, i.e., flow of condensed vapour of circa 10 m³ per hour. In other words, in case of a calamity, substantial amounts of liquid propyne still need to be discharged.

The present invention aims at reducing the instances where liquid propyne is present. Accordingly, the invention relates to a process for the preparation of methacrylate esters, wherein (a) propyne is stripped in a distillation column from a solvent stream containing dissolved propyne, to afford a gaseous propyne stream that is subsequently condensed, and (b) the condensed propyne is contacted with carbon monoxide and an alcohol in the presence of a carbonylation catalyst, characterised in that the propyne is stripped in a distillation column that is equipped with an internal condenser to afford partial condensation for the supply of reflux.

Partial condensation during distillation is known. For instance, on page 1456 of the Chemical Engineers' Handbook, 2nd ed. of 1941, four general methods for the supply of reflux are mentioned: (1) partial condensation of the overhead vapours and return of this partial condensate to the top tray as reflux; (2) total condensation of the overhead vapours and return of a portion of the total condensate to the top tray; (3) combination of 1 and 2; and (4) removal of the hot down flow liquid from the tower, cooling and returning the cooled liquid to the tower. However, it should be noted that in view of the greater cost of condenser surface compared with other means of reflux supply, the inefficiency of such device as a fractionator, and the lack of flexibility and control purposes have caused according to this reference a definite trend away from the use of partial condensation. Similarly, the use of a condenser, i.e., a surface where energy is withdrawn to cause condensation, placed inside a column is also mentioned on page 293 of the textbook "Distillation, Principles and Design Procedures", by RJ Hengstebeck (1966).

In the present process, however, the drawbacks mentioned in the prior art are relatively easily overcome, and anyway largely outweighed by the advantages thereby created.

Conveniently, the internal condenser will condense about two thirds of the vapour stream for supply of reflux. The size of the drum of the overhead condenser may therefore be reduced by a factor three. In addition, the condensed propyne now accumulated in the drum will be of higher purity as any high boiling contaminants (e.g., the solvent) are already condensed in the internal condenser. An example of a suitable distillation column is given on page 206 of volume 3, 4th ed. of Ullmans Encyklopädie der technischen Chemie ("Dampfbeheizter Rieselaustreiber").

Since the reflux is now supplied by the internal condenser, the condensed propyne need not be returned to the column. It may hence be combined with the alcohol it is to be reacted with. In this preferred embodiment, the amount of liquid propyne that is present on-site will be very small. Preferably, the condensed propyne is combined with the alcohol in a molar ratio of 2:1 to 1:2, more preferably of about 1:1 (i.e., reaction stoichiometry).

The amount of liquid (condensing) propyne may be reduced still further by combining the gaseous propyne with the alcohol inside the total overhead condenser. Use of either chilled alcohol or a chilled propyne/alcohol mixture will facilitate condensation of the gaseous propyne the most. This therefore represents the most preferred embodiment of the present invention. Conveniently, the gaseous propyne is brought into contact with an alcohol or a propyne/alcohol mixture chilled to a temperature at least 10 °C below the dew point of propyne, for instance, below -35 °C, preferably below -40 °C.

The C₃-hydrocarbon feeds for the present process may be derived from any fossil carbon processing operation, such as refinery operations. Refinery operations particularly envisaged include ethene crackers; catalytic crackers; and LPG-dehydrogenation processes. For instance, the bottom effluent of a propane/propene splitter is very suitable. Methods for removing heavier hydrocarbons (C₄, etc.) are known and described in EP-A-0,539,628. This document also describes the typical method for isolating propyne (as taught by EP-A-0,392,601). Thus, the concentration of propyne and propadiene may be increased by selective scrubbing the C₃-hydrocarbon feed with a solvent. Suitably, scrubbing is carried out in a column under elevated pressure (2-20 bar gauge, preferably 6-12 barg) using counter current flows. Typically a stream consisting essentially of propane and propene (and less than 0.2% of propyne and propadiene) is removed as the overhead fraction for further use or processing. The solvent which absorbs propyne and, at the elevated pressure employed, also propadiene, suitably comprises a polar organic solvent, such as an amide, e.g., dimethylformamide, dimethylacetamide or N-methylpyrrolidone, a nitrile such as acetonitrile, a sulphone such as sulpholane, or a mixture thereof.

The propadiene is selectively removed, for instance, by extractive distillation. Extractive distillation is a well known method, in which the mixture of propyne and propadiene is dissolved in a polar organic solvent, or available as such a solution, and propadiene is removed as a gas (e.g. by stripping) leaving propyne dissolved in the solvent. Suitable solvents are the same as mentioned herein above.

The propadiene may subsequently be subjected to isomerisation for partial conversion into propyne and be recycled. The isomerisation of propadiene into propyne is well known in the art, and may conveniently be effected in the liquid or gas phase at a temperature in the range of from -30 to 100 °C, preferably 0 to 40 °C, and a pressure in the range of from 0.1 to 100 barg, preferably 1 to 20 barg, in the presence of an isomerisation catalyst, for example a catalyst comprising an alkali metal or alkali metal oxide deposited on alumina, or another isomerisation catalyst as described in Kirk-Othmer's Encyclopaedia of Chemical Technology, 2nd ed., Volume Supplement (1971), pages 547 to 556, and in US-A-3671605.

After selective removal of propadiene, substantially pure propyne can be isolated from the remaining solution by stripping or flashing. The molar concentration in the propyne stream obtained lies above 80%, preferably above 90%, and most preferably above 99%. It is desirable that the molar ratio of propyne to propadiene in the propyne stream is above 100, preferably above 1000, and most preferably above 5000.

The propyne may conveniently be stripped from the solvent in a distillation column operating at a pressure in the range of 0.01 to 5.0 barg, a bottom temperature in the range of 120 to 230 °C and a top temperature in the range of -25 to +35 °C. Preferably, the column is operated at a pressure in the range of 0.1 to 3.0 barg, a bottom temperature in the range of 130 to 210 °C and a top temperature in the range of -15 to +25 °C. Suitably, a column of 6 to 20 theoretical plates is applied. The internal condenser is set to condense in the range of 0.5 to 0.9 of the vapour stream, preferably about two-thirds of the vapour stream. Operating at a convenient reflux of 1 to 20 m³ per hour, energy is withdrawn at a rate of 90 to 2000 kW, for instance of 96 to 1920 kW. The carbonylation catalyst Used in the process according to the invention may be any catalyst having activity for the carbonylation of propyne. It is preferably a Group VIII metal catalyst, more preferably a platinum catalyst, suitably a palladium catalyst.

Preferred groups of catalysts are disclosed in EP-A-0,186,228, EP-A-0,271,144 and EP-A-0,386,833, and include catalysts comprising a palladium compound, a ligand (e.g. a monodentate or bidentate phosphine) and an anion of a weakly or non-coordinating acid.

Specific examples of such catalysts include combinations of (a) palladium acetate, (b) triphenylphosphine, diphenyl-2-pyridylphosphine, or diphenyl(6-methyl-2-pyridyl)phosphine, and (c) p-toluenesulphonic acid, methanesulphonic acid or trifluoroacetic acid.

The alcohol reacted in the carbonylation step of the present process can be any hydroxy-substituted hydrocarbon, which may or may not carry further substituents. The alcohol is preferably an alkanol having up to 20, more preferably 1 to 4 carbon atoms. Examples of alcohols are methanol, ethanol, propanol, isopropanol, n-butanol, isobutanol, and tert-butanol. Most preferably the alkanol is methanol, thus giving methyl methacrylate as the product.

The carbon monoxide reacted in the carbonylation step of the present process, can be derived from any source, and is preferably used in substantially pure form. Minor amounts of inerts or hydrogen may be present, but increase the need for bleed streams and purification of the end product, and therefore decrease the total yield of the present process, if present at too high concentration.

The reaction between propyne, the alcohol and carbon monoxide is preferably effected at a temperature in the range of from 20 to 200 °C, more preferably 20 to 80 °C, and at a pressure in the range of from 5 to 70 barg. Though a separate solvent may be used, for ease of purification it is preferably absent, since the alcohol precursor and methacrylate ester product constitute suitable liquid vehicles for the carbonylation reaction.

The reactants are preferably fed to the process in substantial accordance with the reaction stoichiometry, i.e. in substantially equimolar amounts apart from any recycle streams. The catalyst system is suitably applied at a concentration corresponding to its activity, for example, to produce 25 kg or more of methyl methacrylate per gram of palladium per hour.

The design and operation of the carbonylation unit and further work up equipment is within the skills of a chemical technologist, and does not require further explanation.

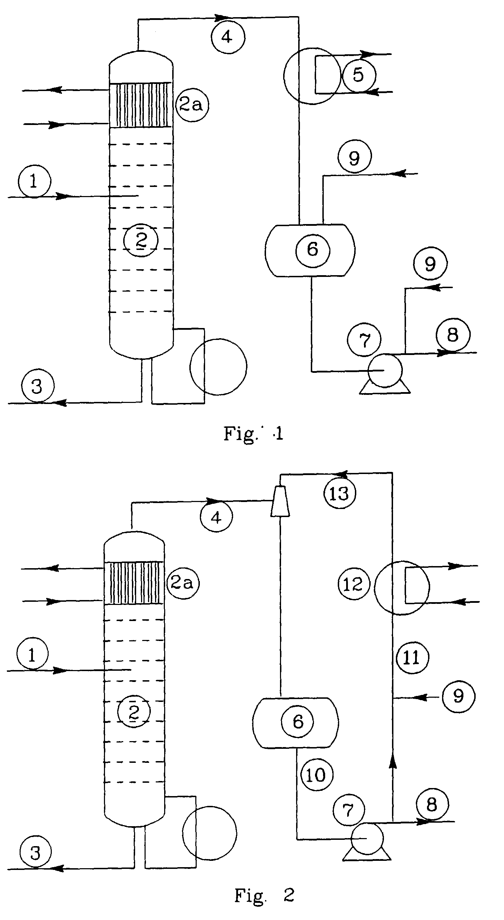

The invention will be further illustrated by the drawings. Herein, Fig. 1 shows a process flow diagram of the process of the invention; wherein propyne is stripped in a distillation column equipped with an internal condenser, as well as some optional modifications that represent preferred embodiments. Fig. 2 shows a process flow diagram wherein the most preferred embodiment of the invention is illustrated.

In Fig. 1, a typical solvent stream (1) comprising 92.9% by weight of DMF and 7.1% by weight of propyne is stripped in a distillation column (2) operating at a feed rate of 42 m³ per hour, a reflux of 5.4 m³ per hour, a pressure of about 1.2 barg and a temperature of -6.5 and 180 °C. The distillation column is equipped with an internal condenser (2a) set to condense about two thirds of the overhead vapour stream (516 kW). The bottoms of this column comprises DMF and is removed through line (3) for reuse. Through line (4) the overhead vapour stream is forwarded to a total overhead condenser (5), wherein propyne is condensed and collected in product accumulator drum (6). Through pump (7) and line (8) the propyne is forwarded to a storage facility or a reactor vessel (not shown). Optionally, as shown in Fig. 1, drum (6) and/or line (8) are provided with an inlet (9) for the alcohol.

In Fig. 2 a process flow diagram similar to Fig. 1 is shown, however, the overhead vapour stream is now combined with a line (13) containing propyne condensate (recycle) in admixture with the alcohol. This stream is drawn off through line (10) from drum (6), combined with the alcohol in line (11), and cooled to -35 °C in heat exchanger (12).

## Claims

1. A process for the preparation of methacrylate esters, wherein (a) propyne is stripped in a distillation column from a solvent stream containing dissolved propyne, to afford a gaseous propyne stream that is subsequently condensed, and (b) the condensed propyne is contacted with carbon monoxide and an alcohol in the presence of a carbonylation catalyst, characterised in that the propyne is stripped in a distillation column that is equipped with an internal condenser to afford partial condensation for the supply of reflux.

2. A process as claimed in claim 1, wherein the gaseous propyne is subsequently condensed in a total overhead condenser and collected in an accumulator drum where it is admixed with the alcohol.

3. A process as claimed in claim 1, wherein the gaseous propyne is subsequently condensed in a total overhead condenser by contact with chilled alcohol or a chilled mixture of alcohol and propyne.

4. A process as claimed in any one of claims 1 to 3, wherein the propyne is stripped from the solvent in a distillation column operating at a pressure in the range of 0.01 to 5.0 barg, a bottom temperature in the range of 120 to 230 °C and a top temperature in the range of -25 to +35 °C.

5. A process as claimed in any one of claims 1 to 4, wherein the propyne is stripped from the solvent in a distillation column operating at a pressure in the range of 0.1 to 3.0 barg, a bottom temperature in the range of 130 to 210 °C and a top temperature in the range of -15 to +25 °C.

6. A process as claimed in any one of claims 1 to 5, wherein the propyne is stripped from the solvent in a distillation column of 6 to 20 theoretical plates.

7. A process as claimed in any one of claims 1 to 6, wherein the internal condenser is set to condense about two-thirds of the vapour stream.

8. A process as claimed in claim 6, wherein the internal condenser operates at a reflux of 1 to 20 m³ per hour, and energy is withdrawn at a rate of 90 to 2000 kW.
